(19) Europäisches Patentamt
European Patent Office
Office européen des brevets

(11) Publication number: **0 376 163 B1**

(12) # EUROPEAN PATENT SPECIFICATION

(45) Date of publication of patent specification: **20.07.94**

(51) Int. Cl.$^5$: **C12N 15/53**, C12N 9/04, C12P 21/02

(21) Application number: **89123676.2**

(22) Date of filing: **21.12.89**

(54) **DNA having lactate oxidase genetic information.**

(30) Priority: **29.12.88 JP 334628/88**

(43) Date of publication of application:
**04.07.90 Bulletin 90/27**

(45) Publication of the grant of the patent:
**20.07.94 Bulletin 94/29**

(84) Designated Contracting States:
**AT BE CH DE ES FR GB GR IT LI LU NL SE**

(56) References cited:
**EP-A- 0 274 425**
**DE-A- 2 755 035**

**PATENT ABSTRACTS OF JAPAN, vol. 10, no. 182 (C-356), 25 June 1986**

**PATENT ABSTRACTS OF JAPAN, vol. 10, no. 345 (C-386), 20 November 1986**

**CHEMICAL ABSTRACTS, vol. 112, no. 1, 1 January-1990, abstract no. 3217t, Columbus, Ohio, US; John D. DUNCAN et al.**

(73) Proprietor: **ASAHI KASEI KOGYO KABUSHIKI KAISHA**
**2-6, Dojimahama 1-chome**
**Kita-ku**
**Osaka(JP)**

(72) Inventor: **Sagai, Hitoshi**
**128-51, Naka**
**Mishima-shi Shizuoka(JP)**
Inventor: **Nogata, Keiko**
**855, Yokamachi**
**Nirayama-cho**
**Tagata-gun**
**Shizuoka(JP)**
Inventor: **Misaki, Hideo**
**852-1, mifuku**
**Ohito-cho**
**Tagata-gun**
**Shizuoka(JP)**

(74) Representative: **Wächtershäuser, Günter, Dr.**
**Tal 29**
**D-80331 München (DE)**

EP 0 376 163 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

Field of the Invention:

A novel DNA encoding a polypeptide constituting a lactate oxidase which is an enzyme catalyzing the reaction producing 1 mole of pyruvic acid and 1 mole of hydrogen peroxide from 1 mole of L-lactic acid and 1 mole of oxygen. The invention also relates to a process for producing lactate oxidase by the use of the DNA gene.

Description of the Background:

Among known lactate oxidases having lactic acid as a substrate reported heretofore are enzymes which catalyze an enzymatic reaction producing 1 mole of pyruvic acid and 1 mole of hydrogen peroxide from 1 mole of L-lactic acid and 1 mole of oxygen according the following reaction scheme:

L-lactic acid + $O_2$ → Pyruvic acid + $H_2O_2$

The enzymes have been known to exist in microorganisms which belong to the genera of *Pediococcus*, *Streptococcus*, *Aerococcus* or the like (Japanese Patent Publication Nos. 4557/1983, 10190/1984, etc.). These lactate oxidases combine with an FAD (flavin adenine dinucleotide) which is a prosthetic group, and express their enzyme activities in a form of a holoenzyme. Since lactate oxidases have lactic acid as their substrate, they can be used for quantitative analysis or purity determination of lactic acid in body fluids such as serum, in various pharmaceutical products containing lactate, in lactic acid fermentation products or in lactic acid reagents. They can also be used for measurements of enzyme activities which are directly or indirectly involved in the production or use of lactic acid, as well as for the elimination of lactic acid in serum. Lactate oxidases are thus very important enzymes.

Besides the lactate oxidases discussed above, there have been other enzymes known by the name "lactate oxidase". They are, for example, L-lactate:oxygen oxidoreductase (Enzyme No. 1.1.3.2) derived from *Mycobacterium phrei* [E. Berman, *Enzyme Handbook*, vol. 1, 111; Splinger-Verlag Berlin Heidelberg, New York, (1969)] or derived from *Mycobacterium avium* [*Nature, 170*, 207 (1952)]. Such an enzyme, different from the lactate oxidase of the present invention, catalyses a reaction producing acetic acid, carbon dioxide and water according to the following formula.

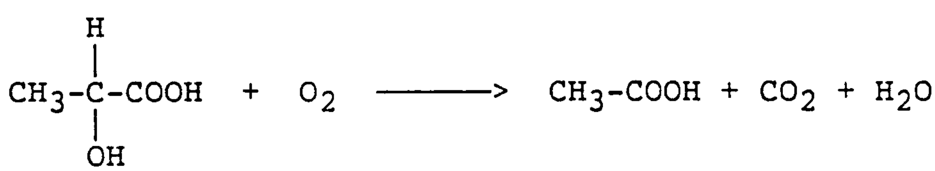

Accordingly, in this invention "lactate oxidase" designates an enzyme catalyzing an enzymatic reaction producing 1 mole of pyruvic acid and 1 mole of hydrogen peroxide from 1 mole of L-lactic acid and 1 mole of oxygen.

Lactate oxidase-producing microorganisms reported heretofore yield only a poor lactate oxidase-producing capability. In addition, a high purification cost is involved in order to obtain a high purity lactate oxidase. The use of these lactate oxidase-producing microorganisms, therefore, have not always been an effective and convenient source for providing lactate oxidase as reagents for abundant use in laboratory experiments or clinical diagnoses.

In addition, there have been no reports concerning the detailed primary structure of a gene coding for an amino acid sequence of lactate oxidase nor about the primary structure of a polypeptide constituting the enzyme.

The present inventors have undertaken extensive studies, including screening of high productivity microorganisms and improvement of lactate oxidase producing stocks, in order to improve the productivity of lactate oxidase and to reduce the production cost. As a result, the inventors have succeeded in determining the primary structure of a DNA gene coding for lactate oxidase and in predicting the primary structure of the polypeptide constituting the enzyme. The inventors further succeeded in obtaining a

transformant possessing a vector into which at least the DNA is inserted. The inventors subsequently succeeded in establishing a process for producing the lactate oxidase by culturing the transformant. Such a finding has led to the completion of the present invention.

## SUMMARY OF THE INVENTION

Accordingly, an object of the present invention is to provide a DNA comprising a base sequence encoding an amino acid sequence of a polypeptide constituting a lactate oxidase which is an enzyme catalyzing the reaction producing 1 mole of pyruvic acid and 1 mole of hydrogen peroxide from 1 mole of L-lactic acid and 1 mole of oxygen.

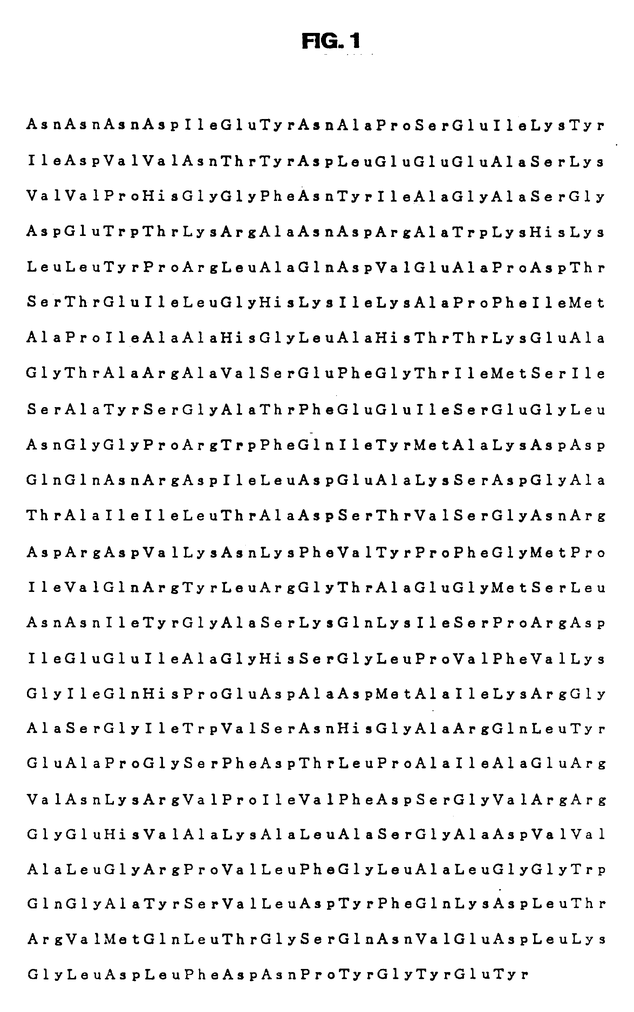

Other objects of this invention are to provide a vector comprising the DNA, a transformant carrying the vector having a DNA which is foreign with respect to a host microorganism, a polypeptide constituting said lactate oxidase and encoding an amino acid sequence given in Figure 1 starting from the N-terminal:

Still another object of this invention is to provide a process for preparing a lactate oxidase comprising: culturing a transformant carrying the vector having a DNA which is foreign with respect to a host microorganism, causing said transformant to express the genetic information of said DNA, and collecting said lactate oxidase from the culture broth.

Other objects, features and advantages of the invention will hereinafter become more readily apparent from the following description.

## BRIEF DESCRIPTION OF THE DRAWING

Figure 1 is the amino acid sequence encoding the polypeptide constituting the lactate oxidase produced in Example 4.

Figure 2 is the base sequence of the DNA produced in Example 4.

Figure 3 is a restriction endoneuclease map of the plasmid pOXI8.

## DETAILED DESCRIPTION OF THE INVENTION AND PREFERRED EMBODIMENTS

The lactate oxidase of this invention causes a novel DNA gene coding for an amino acid sequence of a polypeptide constituting the lactate oxidase (such a DNA is hereinafter referred to as "a lactate oxidase gene" or "a lactate oxidase DNA") to express and to become an active lactate oxidase through coexistence with FDA, which is a prosthetic group. The enzyme has an activity of catalyzing the reaction producing 1 mole of pyruvic acid and 1 mole of hydrogen peroxide from 1 mole of L-lactic acid and 1 mole of oxygen. Several of physicochemical characteristics of the enzyme are listed below.

(a) Action: catalyses the reaction:

L-lactic acid + $O_2$ → Pyruvic acid + $H_2O_2$

(b) Substrate specificity: exhibits a substrate specificity to L-lactic acid.
(c) Optimum pH: about pH 6-7
(d) pH stability: stable at about pH 6.8-8.5
(e) Optimum temperature: about 35°C
(f) Isoelectric point: pH 4.6 ± 0.3 (measured by a focusing electrophoresis using an ampholine carrier)
(g) Molecular weight: 80,000 ± 10,000

The DNA of the present invention can be prepared, for example, by the application of genetic engineering methods. Specifically, the process comprises the following steps. A DNA is first prepared from a lactate oxidase-producing microorganism which is a lactate oxidase gene donor. Following the purification, DNA is treated with ultrasonic waves or with a restriction endoneuclease. This DNA and a linear expression vector DNA are joined using a DNA ligase or the like at the blunt or cohesive ends of the two DNAs to form a closed circle. The recombinant DNA vector thus prepared is introduced into a host microorganism in which the DNA is replicable. Microorganisms having said recombinant DNA vector collected by means of screening using a vector marker or the lactate oxidase activity, or both, as indicators are cultured. The DNA of the present invention possessing lactate oxidase genetic information can be isolated from the recombinant DNA vector which is prepared from the cultured microorganisms.

Any lactate oxidase-producing microorganisms capable of producing lactate oxidase can be used as a lactate oxidase gene-donating microorganism for the purpose of this invention. They are selected from lactate oxidase-producing microorganisms belonging to the genera *Pediococcus*, *Streptococcus*,

*Aerococcus*, and like. Given as specific examples of preferable microorganisms are *Pediococcus* sp. B-0667 (FERM BP-465), *Streptococcus* sp. B-0668 (FERM BP-466), *Streptococcus feacium* ATCC 12755, *Aerococcus viridans* IFO 12219, *Aerococcus viridans* IFO 12317 and the like. A particularly preferable lactate oxidase-producing microorganism is *Aerococcus viridans* IFO 12219.

The method of isolating the DNA derived from the gene-donating microorganism is now exemplified. Any one of the above-mentioned gene-donating microorganisms is first cultured in a liquid culture medium under aeration for 1 to 3 days. The broth thus cultured is subjected to centrifugation to collect the microorganism, which is then lysed to produce a bacteriolysate containing a lactate oxidase gene. The treatment using a cell wall lysing enzyme such as lysozyme or $\beta$-glucanase is used for the bacteriolysis, in combination, as required, with other enzymes such as protease or a surface active agent such as sodium laurylsulfate. In addition, physical destruction of cell walls by means of freeze-thawing or French press, for example, may be employed together with the bacteriolysis.

Conventional methods, including, for example, the deprotein treatment by phenol extraction, protease treatment, ribonuclease treatment, alcohol precipitation, and centrifugation, can be used either independently or in combination for the isolation and purification of DNA from the bacteriolysate.

Digestion of the DNA isolated from the microorganism can be carried out by means of treatment with ultrasonic waves or a restriction endoneuclease, for instance. In order to ensure easy joining of the DNA fragments and the vector DNA, however, the use of a restriction endoneuclease is preferable, especially type II enzymes such as EcoRI, HindIII and BamHI, which can act at specific nucleotide sequences.

Desirable vectors employed are those reconstructed for use as a genetic recombination through artificial treatment of a phage or a plasmid DNA which is capable of replicating autonomously in host bacterial cells.
In cases where *Escherichia coli* is used as a host microorganism, for example, λgt.λC, λgt.λB or the like is used as a phage.

As a plasmid, pBR322, pBR325, pACYC184, pUC12, pUC13, pUC18, pUC19 or the like is used when *Escherichia coli* is a host microorganism; pUB110, pC194 or the like is used when *Bacillus subtillis* is a host microorganism; and YRp7, pYC1, YEp13, pJDB, YIp1 or the like is used when *Saccharomyces cerevisiae* is a host microorganism. In addition, shuttlevectors, which can autonomously replicate in two or more host bacterial cells, for instance, both in *Escherichia coli* and *Saccharomyces cerevisiae*, can be employed. These vectors are desirably digested into linear vector fragments by the use of the same restriction endoneuclease as that used in cleaving the above-mentioned lactate oxidase gene-donating microorganism DNA.

A conventional method of using a DNA ligase can be employed for joining the bacterial DNA and the vector fragment. For instance, cohesive ends of the bacterial DNA and those of the vector fragment are first annealed, and then a recombinant DNA can be prepared from the bacterial DNA fragment and the vector fragment by the action of a suitable DNA ligase. If required, the annealed bacterial DNA-vector fragment is introduced into the host microorganism to produce the recombinant DNA with the aid of intracellular DNA ligase.

Any microorganism which allows autonomic and stable replication of the recombinant DNA and is capable of expressing the character of the foreign DNA can be used as a host bacterium. Examples of such a microorganism include those belonging to *Escherichia coli* such as *Escherichia coli* DH1, *Escherichia coli* HB101, *Escherichia coli* W3110, *Escherichia coli* C600 and the like; those belonging to *Bacillus subtillis* such as *Bacillus subtillis* 207-25 [*Gene*, 34, 1-8 (1985)], *Bacillus subtillis* 207-21 [*Journal of Biochemistry*, 95, 87-93 (1984)], *Bacillus subtillis* BD170 [*Nature*, 293, 481-483 (1981)], *Bacillus subtillis* M (ATCC 6051) and the like; and those belonging to *Saccharomyces cerevisiae* such as *Saccharomyces cerevisiae* AH-22 [*Gene*, 39, 117-120 (1985)], *Saccharomyces cerevisiae* BWG1-7A [*Molecular & Cellular Biology*, 6, 355-367 (1986)] and the like.

Introducing the recombinant DNA into the host microorganism may be performed in the presence of calcium ion when a host microorganism is a bacterium belonging to the genus *Escherichia*. When a bacterium belonging to the genus *Bacillus* is used as a host microorganism, either the competent cell method or the protoplast method can be used. The micro-injection method is also acceptable.

Introducing the objective DNA into the host microorganism can be detected by means of selection of the microorganism which can express a drug resistance marker of the vector on which the objective recombinant DNA is held as well as lactate oxidase activity at the same time. For instance, those bacteria which grow in a selective culture medium of the drug resistance marker and which produce a polypeptide constituting the lactate oxidase can be selected.

The recombinant DNA possessing said lactate oxidase gene once selected in this manner may be easily isolated from the transformant for introduction into another host bacterium. Alternatively, the lactate

oxidase gene DNA can be digested using a restriction endoneuclease or the like from a recombinant DNA possessing a lactate oxidase gene, and is joined with a terminal of other linearized vector obtained in a similar manner. The recombinant DNA with novel characteristics thus prepared is then introduced into other host microorganism.

A DNA coding lactate oxidase mutein which possesses a substantial lactate oxidase activity is a variant gene produced by the genetic engineering technique from a lactate oxidase gene according to this invention. This mutated gene can be obtained by means of various genetic engineering techniques such as the site specific mutagenesis, the substitution of a specific DNA fragment with an artificial mutated gene or the like. Among the lactate oxidase mutein DNAs thus prepared, those having particularly excellent characteristics are finally inserted into a vector to produce a recombinant DNA, which is then introduced into a host microorganism. The lactate oxidase mutein can then be produced.

An example of a specific transformant prepared according to the method discussed above is *Escherichia coli* DH1.pOXI8 (FERM BP-2173). This strain is prepared by inserting a DNA, which is prepared from *Aerococcus viridans* IFO 12219 and coding for lactate oxidase, into plasmid pACYC184, and introducing this plasmid into a host microorganism.

The base sequence of the lactate oxidase gene prepared by the method described above can be determined by the dideoxy method [*Science,* 214, 1205-1210 (1981)].

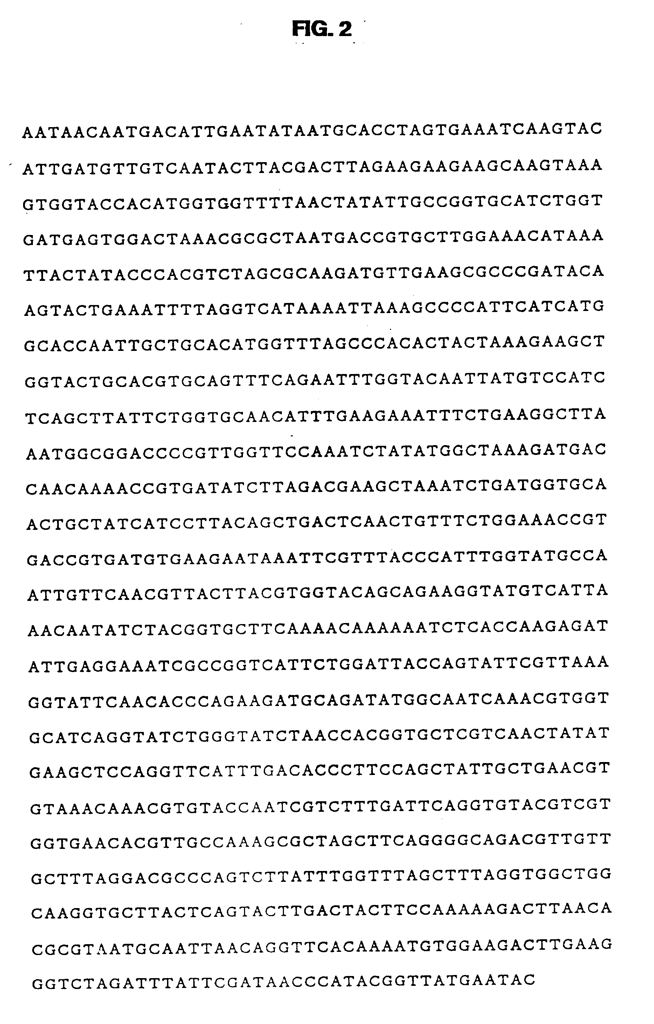

For example, the base sequence of a gene in a plasmid constructed using a microorganism belonging to genus *Aerococcus* as a lactate oxidase gene-donating microorganism and *Escherichia coli* as a host microorganism is represented by Figure 2.

In Figure 2, the upstream nucleotide sequence of the AAT which codes Asn at position 1 of N-terminal, may be any codon for an amino acid, and it may be one or more codons encoding an amino acid. A preferable example is ATG, an initiation codon other than ATG, or a polydeoxyribonucleotide corresponding to a signal peptide. The downstream nucleotide sequence of ATC representing Tyr at the C-terminal may be a translational termination codon or any codon coding for an amino acid. This 3′-end may further have one or more codons encoding an amino acid, provided that in this case it is desirable that an additional translational termination codon be present at the 3′-end of these codons.

The determination of the lactate oxidase DNA of the present invention ensures cloning of the target lactate oxidase-structural gene by the colony hybridization method. According to the method, a DNA fragment encoding the lactate oxidase obtained by the present invention is first extracted, labeled with $^{32}P$ or the like, and subjected to colony hybridization. Cloning of the target lactate oxidase gene can be performed by the selection of a colony carring a plasmid containing the lactate oxidase gene from among the gene library prepared from the lactate oxidase DNA-donating microorganism cells.

The amino acid sequence of the polypeptide produced through the expression of the DNA of this invention can be predicted from the base sequence of the DNA. The amino acid sequence of the portion constituting the N-terminal of said polypeptide can be determined through the method discussed below.

A lactate oxidase gene-donating microorganism capable of producing lactate oxidase is first cultured in a nutrient medium to produce and accumulate lactate oxidase in the cells. The cultured cells are collected from the broth by filtration, centrifugation, or the like means. The collected cells are then destroyed either by a mechanical means or an enzymatic means using lysozyme or the like, and to the lysate EDTA and/or a suitable surface active agent are added, as required, to solubilize and separate lactate oxidase as an aqueous solution. This aqueous solution of lactate oxidase is then condensed or, without being condensed, subjected to ammonium sulfate fractionation, gel filtration, adsorption chromatography, or ion exchange chromatography to obtain a high-purity lactate oxidase. The partial amino acid sequence the N-terminal of this highly purified lactate oxidase is determined using a liquid phase protein sequencer (Beckman System 890ME, manufactured by Beckman, Inc.). In this manner, it was confirmed that the amino acid sequence of said portion was identical to the N-terminal amino acid sequence predicted from analyzing a lactate oxidase gene obtained by a genetic engineering technique. In the amino acid sequence shown in Figure 1, it does not matter that there may be one or more amino acids at upstream of N-terminal Asn. The amino acid sequence determined in this way from the base sequence of Figure 2 is that shown in Figure 1.

Based on the comparison of the molecular weight of the lactate oxidase prepared by the process of the present invention and the amino acid number of the polypeptide of Figure 1, the lactate oxidase of the present invention is assumed to be a dimer of said polypeptide.

A preferred example is a hydrogen atom, a Met or a signal polypeptide. The downstream of Tyr at the C-terminal may be free, or there may be an acid amide or one or more amino acid residues.

The transformant thus obtained, when cultured in a nutrient medium, can produce a large amount of lactate oxidase stably.

Suitable culturing conditions of transformant are determined taking the nutrient-physiological characteristics of the microorganism into consideration. In most cases, liquid culturing is employed. In industrial scale production, however, culturing under deep aerobic stirring is more advantageous. A wide variety of nutrients conventionally used for culturing bacteria can be used for culturing the transformant. Specifically, any nutritious carbon compounds can be used as carbon sources, including, for example, glucose, sucrose, lactose, maltose, fructose, molasses, and the like. As nitrogen sources, any available nitrogen compounds can be employed, including peptones, meat extracts, yeast extracts, casein hydrolysates and the like. Other ingredients, including salts such as phosphates, carbonates, and sulfates, as well as salts of magnesium, calcium, potassium, iron, manganese, zinc and the like, and certain types of amino acids or vitamins, may be used as appropriate.

A culturing temperature may be changed in a range within which the microorganism can grow and produce lactate oxidase. The preferable temperature range is 20-42 °C for *Escherichia coli.* The culturing time may be varied to some degree depending on the culturing conditions. Basically, the culturing is terminated at the time when the yield of lactate oxidase reaches maximum. In usual practice, it takes about 12-48 hours. It is possible to change the pH of the culture media within the range in which the bacteria can grow and produce lactate oxidase. The especially preferable pH range is about 6.0-8.0.

Lactate oxidase may be served for use in the form of culture broth as it contains cells. The lactate oxidase contained in the culture broth, however, is generally used after separation of the cells therefrom by filtration, centrifugation or the like means. When lactate oxidase is contained in the cells, the cells are first separated by means of filtration or centrifugation. The collected cells are then digested either by a mechanical means or an enzymatic means using lysozyme or the like, and to the digested bacteria are added a chelating agent such as EDTA and/or a suitable surface active agent, as required, to solubilize lactate oxidase. Thus, the lactate oxidase is obtained as an aqueous solution.

The solutions containing lactate oxidase thus obtained are then condensed by evaporation under reduced pressure or by the use of a filter, and subjected to a salting-out treatment with ammonium sulfate, sodium sulfate or the like, or to fractional precipitation using a hydrophilic organic solvent such as methanol, ethanol, acetone or the like. The precipitate is dissolved into water, and the solution is dialyzed through a cellulase membrane to eliminate low molecular weight impurities. Alternatively, the precipitate is purified by means of gel filtration, adsorption chromatography, ion-exchange chromatography or the like. A purified lactate oxidase powder is produced from the solution containing lactate oxidase obtained by the use of these various means through vacuum evaporation, lyophilization or the like.

In the description of this specification, amino acids, peptides, nucleic acids, and nucleic acid-related compounds are abbreviated according to the standard prevailing in the field. Some examples of the abbreviations are listed below. Also, all designations of amino acids denote the L-isomers.

| | |
|---|---|
| DNA: | Deoxyribonucleic acid |
| RNA: | Ribonucleic acid |
| A: | Adenine |
| T: | Thymine |
| G: | Guanine |
| C: | Cytosine |
| Ala: | Alanine |
| Arg: | Arginine |
| Asn: | Asparagine |
| Asp: | Aspartate |
| Cys: | Cysteine |
| Gln: | Gluatmine |
| Glu: | Glutamate |
| Gly: | Glycine |
| His: | Histidine |
| Ile: | Isoleucine |
| Leu: | Leucine |
| Lys: | Lysine |
| Met: | Methionine |
| Phe: | Phenylalanine |
| Pro: | Proline |
| Ser: | Serine |
| Thr: | Threonine |
| Trp: | Tryptophan |

Tyr: Tyrosine
Val: Valine

Other features of the invention will become apparent in the course of the following description of the exemplary embodiments which are given for illustration of the invention and are not intended to be limiting thereof.

EXAMPLES

Example 1 [Preparation of Chromosomal DNA]

A chromosomal DNA was prepared from *Aerococcus viridans* (IFO 12219) by the following method. The strain was cultured with shaking in 150 ml of a normal bouillon medium containing 0.5% sodium thiosulfate at 37°C overnight. The culture broth was centrifuged at 3,000 rpm for 10 minutes to collect cells. The cells were suspended into 5 ml of a solution containing 10% sucrose, 50 mM Tris-HCl (pH 8.0), and 50 mM EDTA. To the suspension, 1 ml of a lysozyme solution (10 mg/ml) was added, and the mixture was incubated at 37°C for 15 minutes, followed by the addition of 1 ml of 10% SDS (sodium dodecylsulfate). An equal volume of a mixed solvent of chloroform and phenol (1:1) was added to the suspension, and the mixture was stirred and centrifuged at 10,000 rpm for 3 minutes to separate into water and solvent layers. To the recovered water layer, a 2-fold volume of ethanol was gently added, and the mixture was stirred slowly with a glass rod so as to cause the DNA to wind around the rod. The DNA separated in this manner was dissolved into 10 ml of a solution containing 10 mM Tris-HCl (pH 8.0) and 1 mM EDTA (Such a solution is hereinafter referred to as "TE".). This solution was treated with an equal volume of a chloroform-phenol (1:1) mixed solvent, and was again centrifuged to recover the water layer. A two-fold volume of ethanol was further added to this, and the DNA was again separated from the mixture in the same manner as described above. This finally obtained DNA was dissolved into 2 ml of TE.

Example 2 [Preparation of pACYC184 Plasmid DNA]

*Escherichia coli* pM191 carrying pACYC184 [*J. Bacteriol,* 134, 1141 (1981); ATCC 37033] was cultured with shaking in 1 liter BHI medium (produced by Difco Co.). When the turbidity of the broth reached $OD_{660}$ = 1.0, spectinomycin was added at a final concentration of 300 µg/ml. Shaking of the broth at 37°C was continued for at least 16 hours. Upon termination of the culturing the broth was centrifuged at 3,000 rpm for 10 minutes to collect bacterial cells, from which the plasmid DNA was prepared according to the lysozyme-SDS method and the cesium chloride-ethidium bromide method [Maniatis et al, *Molecular Cloning,* 86-94, Cold Spring Harbor (1982)].

Example 3 [Construction of Plasmid pOXI8 Having Lactate Oxidase Gene]

(1) A mixture was prepared from 2 µl (about 0.5 µg) of *Aerococcus viridans* chromosomal DNA prepared in Example 1, 1 µl of a 10 fold-concentration of EcoRI digestion buffer [500 mM Tris-HCl (pH 7.5), 70 mM $MgCl_2$, 1 M NaCl, and 70 mM mercapto ethanol], 1 µl of EcoRI (10 unit/µl; produced by Takara Shuzo Co., Ltd.), and 6 µl of water. The DNA was digested at 37°C for 1 hour. Separately, plasmid pACYC 184 DNA (about 0.3 µg) was digested with EcoRI in the same manner, and to this was added 0.6 unit of alkaline phosphatase (produced by Takara Shuzo Co., Ltd.; hereinafter referred to as "BAP"). This mixture was incubated at 65°C for 1 hour.

The two solutions of EcoRI-digested DNAs thus prepared were mixed together, and a 0.1 volume of 3M sodium acetate was added to the mixture. Subsequently, the solution was treated with an equal volume of a chloroform-phenol mixed solvent and centrifuged to recover the water layer. To this a 2-fold volume of ethanol was added, and DNA was precipitated by means of centrifugation and dried *in vacuo.* The dried DNA was dissolved into 89 µl of water, and to this were added 10 µl of a 10-fold concentration ligation buffer [0.5 M Tris-HCl (pH 7.6), 0.1 M $MgCl_2$, 0.1 M dithiothreitol, 10 mM spermidine, 10 mM ATP) and 1 µl of T4 DNA ligase (175 unit; produced by Takara Shuzo Co., Ltd.), and the mixture was allowed to stand at 4°C overnight. This DNA solution was treated with chloroform-phenol, and the DNA was precipitated by ethanol, dried *in vacuo,* and dissolved into 10 µl of TE.

(2) *Escherichia coli* DH1 (Stock No. ME8569, ATCC 33849; provided by National Gene Research Institute) which was cultured in 100 ml of BHI medium (Brain Heart Infusion, produced by Difco Co.) and cells were collected at the logarithmic growth phase by centrifugation (10,000 rpm, 2 minutes) and were suspended into 40 ml of an ice-cold solution containing 30 mM potassium acetate, 100 mM RbCl, 10

mM $CaCl_2$, 50 mM $MnCl_2$ and 15% glycerol (pH 5.8). After having been allowed to stand at 0°C for 5 minutes, the suspension was centrifuged to remove the supernatant. The cells were suspended into 4 ml of a solution containing 10 mM MOPS buffer (produced by Dotite Co.), 75 mM $CaCl_2$, 10 mM RbCl and 15% glycerol (pH 6.5), and the suspension was left at 0°C for 15 minutes to obtain competent cells.

(3) To 200 µl of this *Escherichia coli* suspension 10 µl of the DNA solution prepared in (1) above was added. After the mixture was allowed to stand still at 0°C for 30 minutes, 1 ml of BHI medium was added to it. This mixture was kept at 37°C for 90 minutes, a 100 µl aliquot of which was spread over a BHI agar plate containing tetracycline (15 µg/ml), and cultured overnight at 37°C to produce transformants. These transformants were replicated on a lactate oxidase culture plate (composition: peptone, 5 g; NaCl, 1 g; $K_2HPO_4$, 1 g; $MgSO_4$, 0.5 g; peroxidase, 500 IU; dianisidine, 0.1 g; sodium lactate, 5.6 g; agar, 15 g; distilled water, 1 liter; pH 7.0), and were further cultured overnight at 37°C.

Peripheries of four colonies among about 8,000 colonies were colored into charcoal. One of the four strains was named *Escherichia coli* DH1 pOXI8 (FERM BP-2173). After purification, this strain was cultured in a BHI medium overnight at 37°C. The lactate oxidase-activity of the cultured cell measured by the method discussed below was about 0.5 u/ml.

The plasmid carried by the strain was separated in the same manner as in Example 2. This plasmid containing lactate oxidase gene and PACYC184 gene was named pOXI8.

⟨Measurement of Lactate Oxidase Activity⟩

Activity of the lactate oxidase of the present invention was measured according to the following method.

(1) Reagents

First Solution:

| | |
|---|---|
| Peroxidase (50 U/ml) | 0.1 ml |
| 0.2 M 3,3-dimethylglutalate-NaOH buffer (pH 6.5) | 0.2 |
| 15 mM 4-aminoantipyrine | 0.1 |
| 0.5 M DL-lactic acid (pH 6.5) | 0.1 |
| Water | 0.3 |
| Total | 0.8 ml |

Second solution: 0.2% N,N-dimethylaniline
Termination Solution: 0.25% sodium laurylbenzene sulfate

The first solution (0.8 ml) and the second solution (0.2 ml) were mixed and pre-incubated at 37°C for 3 minutes. To this 20 µl of an enzyme solution was added and incubated at 37°C for 10 minutes. Then, 2.0 ml of the termination solution was added to terminate the reaction. The resulting purple color was colorimetrically measured at a wave length of 565 nm. One unit (u) of the enzyme activity is defined as the amount of enzyme which generates 1.0 µmol of $H_2O_2$ per minute at 37°C under the conditions specified in assay procedure. The following equation is followed upon calculation of enzymatic activity (potency) by this measurement method of enzymatic activity.

$$\text{Specific Activity} = \frac{A/10}{35.33 \times 1/2} \times \frac{3.02}{0.02}$$

wherein

A: an increase in optical absorption at 565 nm at 37°C for 10 minutes
10: reaction time (minutes)
35.33: millimolar extinction coefficient of quinonediamine dye ($cm^2/\mu mol$)
1/2: multiplier derived from the fact that 2 mols of $H_2O_2$ produces 1 mol of quinonediamine dye
3.02: final volume (ml)
0.02: volume of enzyme solution (ml)

Example 4 [Mapping of pOXI8 and the Base Sequence Determination]

pOXI8 DNA plasmid was prepared from *Escherichia coli* DH1 pOXI8 in the same manner as the preparation of pACYC184.

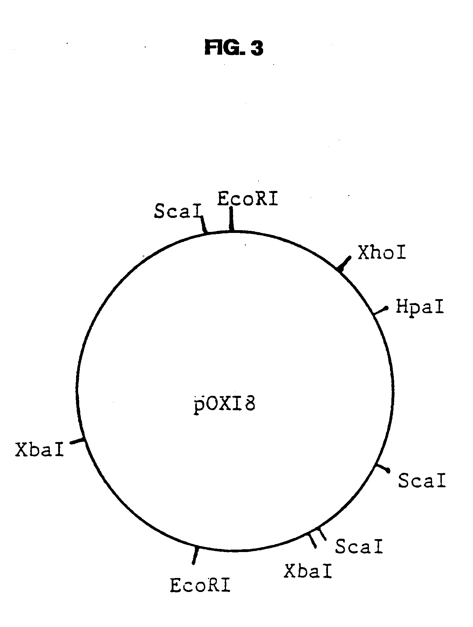

A pOXI8 DNA cleavage map was prepared using restriction endoneucleases EcoRV, HpaI, MluI, ScaI, PstI, XbaI and XhoI (all produced by Takara Shuzo Co., Ltd.). The results are shown in Figure 3. The base sequence of the DNA containing lactate oxidase gene was determined according to the Dideoxy Method using M13 phage [*Science,* 214, 1205 - 1210 (1981)]. The base sequence of lactate oxidase gene and the amino acid sequence of the lactate oxidase are as shown in Figures 2 and 1, respectively.

Example 5 [Production of Lactate Oxidase]

*Escherichia coli* DH1 pOXI8 was cultured in 20 liters of BHI medium at 37°C for 18 hours using a 30-liter jar fermenter. The culture cells were collected by centrifugation at 5,000 rpm for 10 minutes. The cells were washed with 2 liters of physiological saline and suspended into 2 liters of 10 mM phosphate buffer (pH 7.0). To the suspension thus prepared were added 1 mg of lysozyme, 1 ml of 2 mM EDTA-2Na, and TritonX-100 in an amount at a final concentration of 0.1%. After incubation at 37°C for 30 minutes with stirring, the mixture was centrifuged at 5,000 rpm for 10 minutes to separate the resulting supernatant.

To 1.9 l of the supernatant thus obtained was added 30-60% acetone and the precipitate was collected by centrifugation at 5,000 rpm for 30 minutes. The precipitate was dissolved into 200 ml of 10 mM phosphate buffer (pH 7.0, containing 10μM FAD), and subjected to a Sephadex G-25 column for desalting. The desalted solution was subjected to DEAE-Cephallose CL-6B ion exchange chromatography to collect the active fraction. This fraction was desalted and freeze dried to give a powdery product. The lactate oxidase activity measured on this enzyme sample by the method discussed above was 75 u/mg.

Obviously, numerous modifications and variations of the present invention are possible in light of the above teachings. It is therefore to be understood that within the scope of the appended claims, the invention may be practiced otherwise than as specifically described herein.

**Claims**

**1.** A DNA comprising a base sequence encoding an amino acid sequence shown in Figure 1 starting from the N-terminal of a polypeptide constituting a lactate oxidase which is an enzyme catalyzing the reaction producing 1 mole of pyruvic acid and 1 mole of hydrogen peroxide from 1 mole of L-lactic acid and 1 mole of oxygen.

**2.** The DNA according to Claim 1, wherein having the base sequence shown in Figure 2 starting from the 5'-end.

**3.** The DNA according to Claim 1 comprising a base sequence encoding an amino acid sequence shown in Figure 1 starting from the N-terminal of an enzyme having the following physicochemical characteristics:

(a) Action: catalyses the reaction,

$$\text{L-lactic acid} + O_2 \rightarrow \text{Pyruvic acid} + H_2O_2;$$

(b) Substrate specificity: exhibits a substate specificity to L-lactic acid;
(c) Optimum pH: about pH 6.0-7.0;
(d) pH stability: stable at about pH 6.8-8.5.

**4.** A vector comprising the DNA defined in Claim 1.

**5.** A transformant carrying the vector having the DNA of Claim 4, the DNA being foreign with respect to a host microorganism.

**6.** The transformant according to Claim 5, wherein said host microorganism is a microorganism belonging to the genus Escherichia.

**7.** The transformant according to Claim 6, wherein said microorganism belonging to genus Escherichia is Escherichia coli.

**8.** The transformant according to Claim 7, wherein said microrganism belonging to Escherichia coli is Escherichia coli DH1 pOXI8 (Deposited with Fermentation Research Insitute, Agency of Industrial Science and Technology; Deposition No. 2173, FERM BP-2173).

**9.** A polypeptide constituting a lactate oxidase which is an enzyme catalyzing the reaction producing 1 mole of pyruvic acid and 1 mole of hydrogen peroxide from 1 mole of L-lactic acid and 1 mole of oxygen, and having an amino acid sequence shown in Figure 1 stating from the N-terminal.

**10.** A process for the production of a lactate oxidase comprising:
culturing a transformant comprising a vector having the DNA defined in Claim 4, the DNA being foreign with respect to a host microorganism,
causing said transformant to express the genetic information of the DNA defined in Claim 1, and
collecting from the culture broth a lactate oxidase which is an enzyme catalyzing the reaction producing 1 mole of pyruvic acid and 1 mole of hydrogen peroxide from 1 mole of L-lactic acid and 1 mole of oxygen.

**11.** The process according to Claim 10, wherein said lactate oxidase has the following physicochemical characteristics:
(a) Action: catalyses the reaction,

L-lactic acid + $O_2$ → Pyruvic acid + $H_2O_2$;

(b) Substrate specificity: exhibits a substrate specificity to L-lactic acid;
(c) Optimum pH: about pH 6.0 - 7.0;
(d) pH stability: stable at about pH 6.8-8.5.

**12.** The process according to Claim 10, wherein said lactate oxidase comprises as its constituent a polypeptide having an amino acid sequence shown in Figure 1 starting from the N-terminal.

**Patentansprüche**

**1.** DNA, umfassend eine Basensequenz, die für eine in Figur 1 dargestellte Aminosäuresequenz kodiert, ausgehend vom N-terminalen Ende eines Polypeptids, das eine Lactat-Oxidase darstellt, die ein Enzym ist, das die Reaktion, bei der aus 1 Mol 1-Milchsäure und 1 Mol Sauerstoff 1 Mol Brenztraubensäure und 1 Mol Wasserstoffperoxid hergestellt werden, katalysiert.

**2.** DNA nach Anspruch 1 mit der Basensequenz, dargestellt in Figur 2, ausgehend vom 5'-Ende.

**3.** DNA nach Anspruch 1, umfassend eine Basensequenz, die für eine in Figur 1 dargestellte Aminosäuresequenz kodiert, ausgehend vom N-terminalen Ende eines Enzyms mit den nachstehenden physikochemischen Eigenschaften:
(a) Wirkung: katalysiert die Reaktion,

L-Milchsäure + $O_2$ → Brenztraubensäure + $H_2O_2$

(b) Substratspezifität: zeigt eine Substratspezifität für L-Milchsäure;
(c) Optimaler pH-Wert: etwa pH 6,0-7,0
(d) pH-Stabilität: stabil bei etwa pH 6,8-8,5.

**4.** Vektor, umfassend die DNA gemäß Anspruch 1.

**5.** Transformante, die den Vektor mit der DNA nach Anspruch 4 trägt, wobei die DNA hinsichtlich eines Wirtsmikroorganismus fremd ist.

6. Transformante nach Anspruch 5, wobei der Wirtsmikroorganismus ein Mikroorganismus, der zur Gattung Escherichia gehört, ist.

7. Transformante nach Anspruch 6, wobei der Mikroorganismus, der zur Gattung Escherichia gehört, Escherichia coli ist.

8. Transformante nach Anspruch 7, wobei der Mikroorganismus, der zu Escherichia coli gehört, Escherichia coli DH1 pOXI8 ist (hinterlegt beim Fermentation Research Institute, Agency of Industrial Science and Technology; Hinterlegungsnummer 2173, FERM BP-2173).

9. Polypeptid, das eine Lactat-Oxidase ausmacht, die ein Enzym ist, das die Reaktion, bei der aus 1 Mol L-Milchsäure und 1 Mol Sauerstoff 1 Mol Brenztraubensäure und 1 Mol Wasserstoffperoxid hergestellt werden, katalysiert und das eine Aminosäuresequenz wie in Figur 1 aufweist, ausgehend vom N-terminalen Ende.

10. Verfahren zur Herstellung einer Lactat-Oxidase, umfassend:
Züchten einer Transformante, umfassend einen Vektor mit der DNA nach Anspruch 4, wobei die DNA hinsichtlich des Wirtsmikroorganismus fremd ist,
Veranlassen der Transformante, die genetische Information der in Anspruch 1 definierten DNA zu exprimieren und
Gewinnen aus der Kulturbrühe eine Lactat-Oxidase, die ein Enzym ist, das die Reaktion, bei der aus 1 Mol L-Milchsäure und 1 Mol Sauerstoff 1 Mol Brenztraubensäure und 1 Mol Wasserstoffperoxid hergestellt werden, katalysiert.

11. Verfahren nach Anspruch 10, wobei die Lactat-Oxidase die nachstehenden physikochemischen Eigenschaften aufweist:
(a) Wirkung: katalysiert die Reaktion,

L-Milchsäure + $O_2$ → Brenztraubensäure + $H_2O_2$

(b) Substratspezifität: zeigt eine Substratspezifität für L-Milchsäure;
(c) Optimaler pH-Wert: etwa pH 6,0-7,0
(d) pH-Stabilität: stabil bei etwa pH 6,8-8,5.

12. Verfahren nach Anspruch 10, wobei die Lactat-Oxidase als Bestandteil ein Polypeptid mit einer Aminosäuresequenz, gezeigt in Figur 1, ausgehend vom N-terminalen Ende, umfaßt.

**Revendications**

1. ADN comprenant une séquence de base encodant une séquence acide aminé montrée en figure 1 démarrant du N-terminal d'un polypeptide constituant une lactate oxydase qui est un enzyme catalysant la réaction produisant 1 mole d'acide pyruvique et 1 mole de peroxyde d'hydrogène à partir d'l mole d'acide L-lactique et d'1 mole d'oxygène.

2. ADN selon la revendication 1, dans lequel il y a la séquence de base montrée en figure 2 démarrant de l'extrémité 5'.

3. ADN selon la revendication 1, comprenant une séquence de base encodant une séquence acide aminé montrée en figure 1 démarrant du N-terminal et un enzyme ayant les caractéristiques physicochimiques suivantes
(a) Action : catalyse la réaction :

acide L-lactique + $O_2$ → acide pyruvique + $H_2O_2$

(b) Spécificité de substrat : montre une spécificité de substrat à l'acide L-lactique.
(c) pH optimum : environ pH 6,0-7,0.
(d) Stabilité au pH : stable à environ pH 6,8-8,5.

**4.** Vecteur comprenant l'ADN défini en revendication 1.

**5.** Transformant portant le vecteur ayant l'ADN de la revendication 4, l'ADN étant étranger par rapport à un microorganisme hôte.

**6.** Transformant selon la revendication 5, dans lequel ledit microorganisme hôte est un microorganisme appartenant au gène Escherichia.

**7.** Transformant selon la revendication 6, dans lequel ledit microorganisme appartenant au gène Escherichia est Escherichia coli.

**8.** Transformant selon la revendication 7, dans lequel ledit microorganisme appartenant à Escherichia coli est Escherichia coli DH1 pOXI8 (déposé auprès du Fermentation Research Institute, Agency of Industrial Science and Technology, N° de dépôt 2173, FERM BP-2173).

**9.** Polypeptide constituant une lactate oxydase qui est un enzyme catalysant la réaction produisant 1 mole d'acide pyruvique et 1 mole de peroxyde d'hydrogène à partir d'1 mole d'acide L-lactique et 1 mole d'oxygène, et ayant une séquence acide aminé montrée en figure 1 démarrant du N-terminal.

**10.** Procédé pour la préparation d'une lactate oxydase comprenant les étapes de :
cultiver un transformant comprenant un vecteur ayant l'ADN défini en revendication 4, l'ADN étant étranger par rapport à un microorganisme hôte,
faire que ledit transformant exprime l'information génétique de l'ADN défini en revendication 1, et
collecter du bouillon de culture une lactate oxydase qui est un enzyme catalysant la réaction produisant 1 mole d'acide pyruvique et 1 mole de peroxyde d'hydrogène à partir d'1 mole d'acide L-lactique et d'1 mole d'oxygène.

**11.** Procédé selon la revendication 10, dans lequel ladite lactate oxydase a les caractéristiques physicochimiques suivantes :
(a) Action : catalyse la réaction :

acide L-lactique + $O_2$ → acide pyruvique + $H_2O_2$

(b) Spécificité de substrat : montre une spécificité de substrat à l'acide L-lactique.
(c) pH optimum : environ pH 6,0-7,0.
(d) Stabilité au pH : stable à environ pH 6,8-8,5.

**12.** Procédé selon la revendication 10, dans lequel ladite lactate oxydase comprend comme son constituant un polypeptide ayant une séquence acide aminé montrée en figure 1 démarrant du N-terminal.

# FIG. 1

AsnAsnAsnAspIleGluTyrAsnAlaProSerGluIleLysTyr
IleAspValValAsnThrTyrAspLeuGluGluGluAlaSerLys
ValValProHisGlyGlyPheAsnTyrIleAlaGlyAlaSerGly
AspGluTrpThrLysArgAlaAsnAspArgAlaTrpLysHisLys
LeuLeuTyrProArgLeuAlaGlnAspValGluAlaProAspThr
SerThrGluIleLeuGlyHisLysIleLysAlaProPheIleMet
AlaProIleAlaAlaHisGlyLeuAlaHisThrThrLysGluAla
GlyThrAlaArgAlaValSerGluPheGlyThrIleMetSerIle
SerAlaTyrSerGlyAlaThrPheGluGluIleSerGluGlyLeu
AsnGlyGlyProArgTrpPheGlnIleTyrMetAlaLysAspAsp
GlnGlnAsnArgAspIleLeuAspGluAlaLysSerAspGlyAla
ThrAlaIleIleLeuThrAlaAspSerThrValSerGlyAsnArg
AspArgAspValLysAsnLysPheValTyrProPheGlyMetPro
IleValGlnArgTyrLeuArgGlyThrAlaGluGlyMetSerLeu
AsnAsnIleTyrGlyAlaSerLysGlnLysIleSerProArgAsp
IleGluGluIleAlaGlyHisSerGlyLeuProValPheValLys
GlyIleGlnHisProGluAspAlaAspMetAlaIleLysArgGly
AlaSerGlyIleTrpValSerAsnHisGlyAlaArgGlnLeuTyr
GluAlaProGlySerPheAspThrLeuProAlaIleAlaGluArg
ValAsnLysArgValProIleValPheAspSerGlyValArgArg
GlyGluHisValAlaLysAlaLeuAlaSerGlyAlaAspValVal
AlaLeuGlyArgProValLeuPheGlyLeuAlaLeuGlyGlyTrp
GlnGlyAlaTyrSerValLeuAspTyrPheGlnLysAspLeuThr
ArgValMetGlnLeuThrGlySerGlnAsnValGluAspLeuLys
GlyLeuAspLeuPheAspAsnProTyrGlyTyrGluTyr

FIG. 2

AATAACAATGACATTGAATATAATGCACCTAGTGAAATCAAGTAC
ATTGATGTTGTCAATACTTACGACTTAGAAGAAGAAGCAAGTAAA
GTGGTACCACATGGTGGTTTTAACTATATTGCCGGTGCATCTGGT
GATGAGTGGACTAAACGCGCTAATGACCGTGCTTGGAAACATAAA
TTACTATACCCACGTCTAGCGCAAGATGTTGAAGCGCCCGATACA
AGTACTGAAATTTTAGGTCATAAAATTAAAGCCCCATTCATCATG
GCACCAATTGCTGCACATGGTTTAGCCCACACTACTAAAGAAGCT
GGTACTGCACGTGCAGTTTCAGAATTTGGTACAATTATGTCCATC
TCAGCTTATTCTGGTGCAACATTTGAAGAAATTTCTGAAGGCTTA
AATGGCGGACCCCGTTGGTTCCAAATCTATATGGCTAAAGATGAC
CAACAAAACCGTGATATCTTAGACGAAGCTAAATCTGATGGTGCA
ACTGCTATCATCCTTACAGCTGACTCAACTGTTTCTGGAAACCGT
GACCGTGATGTGAAGAATAAATTCGTTTACCCATTTGGTATGCCA
ATTGTTCAACGTTACTTACGTGGTACAGCAGAAGGTATGTCATTA
AACAATATCTACGGTGCTTCAAAACAAAAAATCTCACCAAGAGAT
ATTGAGGAAATCGCCGGTCATTCTGGATTACCAGTATTCGTTAAA
GGTATTCAACACCCAGAAGATGCAGATATGGCAATCAAACGTGGT
GCATCAGGTATCTGGGTATCTAACCACGGTGCTCGTCAACTATAT
GAAGCTCCAGGTTCATTTGACACCCTTCCAGCTATTGCTGAACGT
GTAAACAAACGTGTACCAATCGTCTTTGATTCAGGTGTACGTCGT
GGTGAACACGTTGCCAAAGCGCTAGCTTCAGGGGCAGACGTTGTT
GCTTTAGGACGCCCAGTCTTATTTGGTTTAGCTTTAGGTGGCTGG
CAAGGTGCTTACTCAGTACTTGACTACTTCCAAAAAGACTTAACA
CGCGTAATGCAATTAACAGGTTCACAAAATGTGGAAGACTTGAAG
GGTCTAGATTTATTCGATAACCCATACGGTTATGAATAC

FIG. 3